# EUROPEAN PATENT SPECIFICATION

(11) **EP 2 772 138 B1**
(45) Date of publication and mention of the grant of the patent: **19.04.2017**
(21) Application number: 14425019.8
(22) Date of filing: 26.02.2014
(51) Int. Cl.: A01N 59/00, A61L 2/20, A01P 1/00

(54) **Method to remove microorganisms on plant material**
Verfahren zur Entfernung von Mikroorganismen auf Pflanzenmaterial
Procédé pour éliminer des micro-organismes sur un matériel végétal

(30) Priority: 01.03.2013 IT RM20130124
(43) Date of publication of application: 03.09.2014
(73) Proprietor: Jingold S.P.A., 47522 Cesena (FC) (IT)
(72) Inventor: Balestra, Giorgio Mariano, 01027 Montefiascone (VT) (IT)
(74) Representative: Nesci, Lucia

(56) References cited:
- DE-A1- 3 541 706
- DE-A1- 3 917 250
- DATABASE WPI Week 201082 Thomson Scientific, London, GB; AN 2010-N68661 XP002712577, & KR 2010 0115659 A (UNIV CHONNAM NAT IND FOUND) 28 October 2010 (2010-10-28)
- MELTEM YESILCIMEN AKBAS ET AL: "Effectiveness of ozone for inactivation of Escherichia coli and Bacillus cereus in pistachios", INTERNATIONAL JOURNAL OF FOOD SCIENCE AND TECHNOLOGY, vol. 41, no. 5, 1 May 2006 (2006-05-01), pages 513-519, XP055078597, ISSN: 0950-5423, DOI: 10.1111/j.1365-2621.2005.01099.x
- M. Prabakaran ET AL: "Effect of Ozonization on pathogenic bacteria", Advances in applied Science Research, 1 January 2012 (2012-01-01), pages 299-302, XP055078607, Retrieved from the Internet: URL:http://pelagiaresearchlibrary.com/adva nces-in-applied-science/vol3-iss1/AASR-201 2-3-1-299-302.pdf [retrieved on 2013-09-10]
- G. M. BALESTRA ET AL: "Pseudomonas syringae pv. syringae Causal Agent of Disease on Floral Buds of Actinidia deliciosa (A. Chev) Liang et Ferguson in Italy", JOURNAL OF PHYTOPATHOLOGY - PHYTOPATHOLOGISCHE ZEITSCHRIFT., vol. 145, no. 8-9, 1 September 1997 (1997-09-01), pages 375-378, XP055319238, DE ISSN: 0931-1785, DOI: 10.1111/j.1439-0434.1997.tb00417.x

## Description

The present invention concerns a methodology that can effectively eliminate the microbial load characterised by bacterial cells of *Pseudomonas syringae* pv. *actinidiae* (PSA), that are a causative agent of bacterial canker of actinidia. This is an extremely virulent bacterium which attacks all species and varieties of Actinidia.

The methodology described is applicable to different plants of the Genus *Actinidia* to prevent the serious consequences of the presence and spread of the bacterium; specifically the methodology is applied to actinidia pollen potentially infected by PSA.

The state of current techniques adopted against Pseudomonas syringae pv. actinidiae (PSA) involves the use of different strategies that are applied individually and/or in combination:
a) to provide proper nourishment and stimulate the endogenous defences of Actinidia plants (administration of amino acids, potassium humates, phosphorous acid, sulphuric acid (Mazzaglia et al., 2011); b) to protect the different phenological phases of the actinidia by copper salts (Fratarcangeli et al., 2010; Quattrucci: et al., 2010); c) to prevent infection by the causal agent of bacterial canker of actinidia by using a natural antagonist (Bacillus amyloliquefaciens, strain D747), which is the only phytosanitary product to date that has been registered in the European Community to combat PSA, which can colonize and protect the organs of actinidia plants against this bacterium (http://www.intrachem.it/home.cfm?lang=it&section=3&news=15).

DE 39 17 250 A1 discloses a method of sanitizing vegetables, spices, cereals, fruits etc. that uses ozone as a sterilizing agent and comprises two phases. During the first one the material to be sterilized is kept for ten hours under a sterile atmosphere with 10-16% humidity at 20-40 °C. During the second phase the material is treated for 8 hours under a stream of ozone and carbon dioxide. Flowers of chamomile are treated in such a way in one example. The embodiments given by DE 3917 250 describe the use of CO₂ (carbon dioxide) in a mixture with O₃ (ozone) and all the specific claims relate to their exclusive use in mixture.

DE 33 255 68A1 relates to the sterilization of bulk goods in one phase. DATABASE WPI Week 201082 (Thompson Scientific, London, GB) relates to the production of antibiotics by a. *Pseudomonas aeruginosa* NO3 and their subsequent employment against another bacterium, i.e. *Xanthomonas citri.*

Meltam Yesilcimen Akbas et al: "Effectiveness of ozone for inactivation of Escherichia cnli and Bacillus cereus in pistachios" International Journal of Food Science and Tecnology, vol. 41, no. 5, 1 May 2006, pages 513-519, relates to pistachio fruits; the treatment is carried out with ozone at concentration levels and times different from those of the invention. *Escherichia coli* and *bacillus cereus* are bacterial species quite different from those in the present invention and are studied in relation to exclusive damages to human health. Balestra et al: "Pseudomonas syringae pv. syringae causal agent of disease on floral buds of Actinidia deliciosa Liang et Ferguson in Italy" J. Pathology 145, causal agent of disease on floral buds of Actinidia deliciosa Liang et Ferguson in Italy" J. Pathology 145, 375-378 (1997), describes the bacterial infection of kiwi plants and how the treatment is limited to pre-emergence application of cupric products. Therefore, the object of this invention is to identify a methodology that, when applied to plants of the genus Actinidia, is able to eliminate the microbial load characterised by PSA bacterial cells, by using ozone in combined bactericidal activity and, subsequently, temperature.

Book references: MAZZAGLIA A., RENZI M., TARATUFOLO M. C., ROSSETTI A., BALESTRA G. M. (2011). Tecniche di campo e nutrizione contro il cancro del kiwi). L'Informatore Agrario 10, 64-66*;* FRATARCANGELI L, ROSSETTI A, MAZZAGLIA A, BALESTRA G.M. (2010). Il ruolo del rame nella lotta al cancro batterico del kiwi. INFORMATORE AGRARIO, vol. 8; p. 52-55, ISSN: 0020689*;* QUATTRUCCI A, KENZI M, ROSSETTI A, RICCI L, TARATUFOLO C, MAZZAGLIA A, BALESTRA G.M. (2010). Cancro batterico del kiwi verde: nuove strategie di controllo. L'informatore agrario 16: 53-58*.* INFORMATORE AGRARIO, vol. 16; p. 53-58, ISSN: 0020-0689*.*

A detailed description now follows to illustrate the features and advantages of this invention according to which the methodology of elimination of the microbial load characterised by the bacterial cells of *Pseudomonas syringae* pv. *actinidiae* (PSA), that are causative agent of bacterial canker of actinidia, is applied to a plant of the genus Actinidia, specifically to a sample of pollen potentially infected by PSA; this is given just by way of example for all the many different fields of application of the invention.

The method involves two phases.
Phase 1 is a non-chemical process in which Actinidia pollen sample is subjected to a specific temperature (42 ° C) for a given period of time (30 minutes), while the same sample is stirred by a suitable air flow. An antimicrobial action is exerted on the treated sample of Actinidia pollen by using a specific temperature for a defined period of time.

In phase 2 the plant sample already subjected to phase 1, still stirred by a suitable air flow, is treated with an odourless and colourless substance (ozone). A specific concentration of ozone is used (0.05 ppm) for a given period of time (4 hours), and in specific environmental conditions: temperature of 5°C and a relative air humidity (RH) comprised between given values and no more than 35%.

If used separately on the same plant sample, the antimicrobial effect of the two phases (1 and 2) is lower.

The above methodology, in which the two phases (phase 1 and phase 2) are applied in succession, increases the bactericidal action of temperature and ozone, if other parameters (treatment duration, temperature and % RH - relative humidity - in the presence of ozone), which are essential for achieving the desired object, are taken in due account.

As regards the treatment of pollen samples, an ad hoc system could be developed, for example, to treat lots of refrigerated young actinidia vines before they are planted in open field.

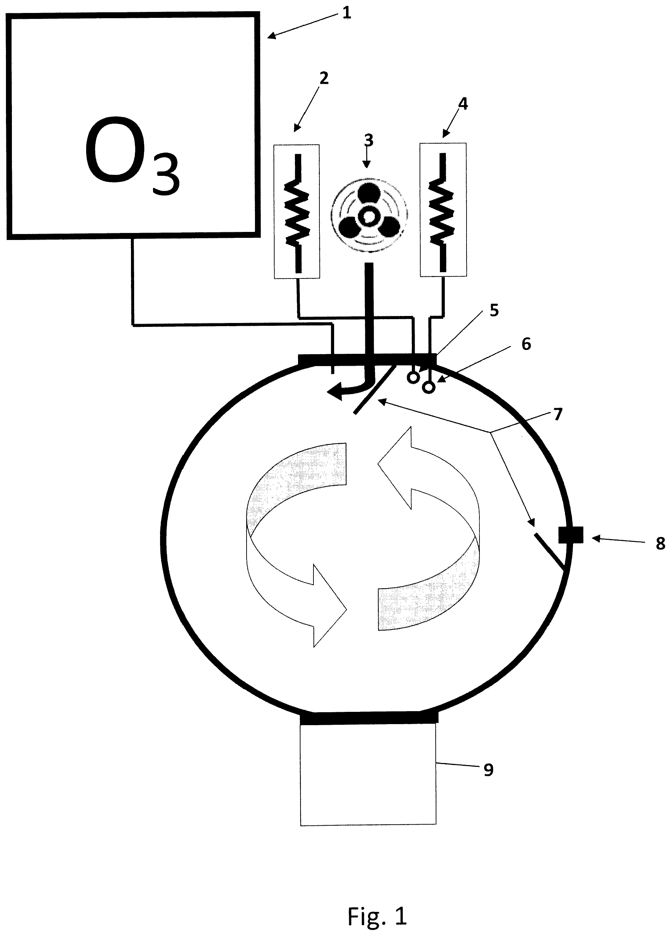

Fig. 1 shows the application of the methodology that is the object of this invention.

In the prototype, a sample of Actinidia pollen, potentially infected by PSA, is placed inside a container equipped with an ozone generator 1, a cooler 2, a fan 3, a radiator 4, a cold temperature probe 5, a hot temperature probe 6, protection flaps 7 that prevent the pollen to stick to protruding parts inside the same container, an opening for measuring by test tubes 8, a drawer 9 to input the pollen and collect it after it has been treated.

In the first phase of the methodology, the Actinidia pollen inside the container is stirred by a suitable air flow and is subjected to a temperature of 42°C for 30 minutes. In phase 2 of the methodology, the Actinidia pollen is treated with ozone (O₃), at a specific concentration (0.05 ppm), for 4 hours, at a temperature of 5°C and a humidity lower than a maximum value of 35%.

## Claims

1. A methodology for eliminating the microbial load of the bacterial cells of *Pseudomonas syringae* pv. *Actinidiae* (PSA), that are causative agents of the bacterial canker of the actinidia, methodology which is applicable to different plants of the genus Actinidia, **characterised in that** the methodology comprises two successive phases as an antibacterial treatment of *Actinidia* spp. pollen, the first phase of which consisting of subjecting a plant sample to a specific temperature and for a given period of time, and the second phase consisting of using ozone on the same sample at a specific concentration and within specific environmental values of temperature and relative air humidity.

2. The methodology according to claim 1, wherein in the first phase said pollen of the genus *Actinidia* is subjected to a temperature of 42 °C for 30 minutes.

3. The methodology according to claim 1, wherein in the second phase said pollen of the genus *Actinidia* is subjected to ozone at a specific concentration of 0.05 ppm for 4 hours, at a temperature of 5 °C and a maximum relative air humidity of 35%.

4. The methodology according to claims 1 to 3, wherein the treatments applied in succession (temperature and ozone) are carried out in a confined environment where air relative humidity, temperature, ozone concentration, air flow have to be controlled.

## Patentansprüche

1. Methodologie zum Entfernen von mikrobieller Belastung der Bakterienzellen von *Pseudomonas syringae* pv. *Actinidiae* (PSA), die Erreger von bakteriellem Baumkrebs der Actinidia sind, Methodologie welche sich an unterschiedliche Pflanzen der *Actinidia* Klasse anwenden lässt, **dadurch gekennzeichnet, dass** die Methodologie zwei sukzessiven Phasen umfasst als antibakterielle Behandlung des *Actinidia* spp. Pollen, wobei deren erste Phase aus einer Pflanzenprobeaussetzung bei einer spezifischen Temperatur und für einen gegebenen Zeitraum besteht, und die zweite Phase aus der Verwendung von Ozon auf der gleichen Probe bei einer spezifischen Konzentration und innerhalb spezifischen Umgebungswerte von Temperatur und relative Luftfeuchtigkeit besteht.

2. Methodologie nach Anspruch 1, wobei in der ersten Phase der Pollen der *Actinidia* Klasse einer Temperatur von 42°C für 30 Minuten ausgesetzt ist.

3. Methodologie nach Anspruch 1, wobei in der zweiten Phase der Pollen der *Actinidia* Klasse dem Ozon bei einer spezifischen Konzentration von 0.05 ppm für 4 Stunden, bei einer Temperatur von 5°C und bei einer maximalen relativen Luftfeuchtigkeit von 35% ausgesetzt ist.

4. Methodologie nach Anspruch 1 bis 3, wobei die nacheinander angebrachte Behandlungen (Temperatur und Ozon) in einer eingeschränkten Umgebung durchgeführt werden, wo relative Luftfeuchtigkeit, Temperatur, Ozonkonzentration, Luftstrom kontrolliert werden müssen.

## Revendications

1. Méthodologie servant à éliminer la charge microbienne de cellules bactériennes de *Pseudomonas syringae* pv. *Actinidiae* (PSA), étant des agents causals du flétrissement bactérien de l'actinidia, méthodologie étant applicable à différentes plantes du genre Actinidia, **caractérisée en ce que** la méthodologie comprend deux phases successives comme traitement antibactérien du pollen d'*Actinidia* spp., dont la première phase consiste à exposer un échantillon de plante à une température spécifique et pendant une période de temps définie, et la seconde phase consiste à utiliser de l'ozone sur le même échantillon à une concentration spécifique et selon une fourchette de valeurs environnementales spécifiques de température et d'humidité relative de l'air.

2. Méthodologie selon la revendication 1, dans laquelle dans la première phase ledit pollen du genre *Actinidia* est exposé à une température de 42°C pendant 30 minutes.

3. Méthodologie selon la revendication 1, dans laquelle dans la seconde phase ledit pollen du genre *Actinidia* est exposé à l'ozone à une concentration spécifique de 0,05 ppm pendant 4 heures, à une température de 5°C et une humidité relative de l'air maximale de 35 %.

4. Méthodologie selon les revendications 1 à 3, dans laquelle les traitements appliqués successivement (température et ozone) sont exécutés dans un environnement confiné où l'humidité relative de l'air, la température, la concentration d'ozone et le débit d'air doivent être contrôlés.
